# EUROPEAN PATENT APPLICATION

(11) **EP 3 336 597 A1**
(43) Date of publication of application: **20.06.2018**
(21) Application number: 16204933.2
(22) Date of filing: 19.12.2016
(51) Int. Cl.: G02B 21/00, A61B 3/15, A61B 3/125

(54) **MICROSCOPE OR ENDOSCOPE ASSEMBLY AND METHOD FOR REDUCING SPECULAR REFLECTIONS**

(71) Applicant: Leica Instruments (Singapore) Pte. Ltd., 608924 Singapore (SG)
(72) Inventor: Themelis, George, 88131 Lindau (DE)
(74) Representative: Grünecker Patent- und Rechtsanwälte PartG mbB

(57) **Abstract**

The invention relates to a microscope or endoscope assembly (1), in particular for a surgical microscope such as an ophthalmic microscope. Especially in eye surgery, but also in other application concerning life tissue, specular reflections of the illumination light are unwanted because they may hide important information contained in a diffuse reflection at the same location. In order to suppress such unwanted specular reflection, the microscope or endoscope assembly according to the invention comprises an observation region (4), in which an object (6) to be observed, such as an eye, can be arranged. The assembly further comprises an illumination light path (8) which extends from an illumination light entry region (24), where illumination light enters into the assembly, to the observation region. Further, an observation light path (10) is comprised, which extends from the observation region (4) to a light exit region (34), where the light leaves the assembly and may be collected by an observation subassembly (36), such as at least one camera and/or at least one ocular. In the illumination light path, a first polarizing subassembly (16) is arranged, to create polarized illumination light (18). In the observation light path (10) a second polarizing subassembly (32) is arranged, which is configured to filter out the polarized illumination light (18) passing the first polarizing subassembly (16). To ensure coaxial illumination and observation of the object, a beam splitter (30, 40) is provided, which is arranged in the illumination light path (8) between the light entry region (24) and the observation region (4) and in the observation light path (10) between the observation region (4) and the light exit region (34). By the complementary filtering action of the first and second polarizing subassembly, specular reflections by the object can be suppressed and/or eliminated.

## Description

The invention relates to a microscope or an endoscope assembly, in particular for a surgical microscope such as an ophthalmic microscope. The invention further relates to a retrofit kit for generating such a microscope assembly and to the use of such a microscope assembly as well as to a method for operating a microscope, in particular a surgical microscope.

Ophthalmic microscopes are used for operations on the eye, such as cataract surgery. In cataract surgery, ophthalmologists rely on the red reflex to see the capsule, lens and anterior chamber structure of the eye. The red reflex is, in essence, a total light reflection within the eye, which can be achieved when the eye is illuminated from the right angle and from the right distance. The red reflex acts as a background illumination for the observation of the lens of the eye. However, the illumination used for the red reflex causes strong lightening artifacts called Purkinje reflexes, which are essentially specular reflections. The Purkinje reflections can be so intense that the view into the eye is obstructed and the observed image contrast is reduced.

The same problem also exists in other surgical microscopes which are used for other applications. If the tissue is illuminated, specular reflections may be generated which deteriorate the image quality.

Specular reflection corresponds to a mirror-like reflection of the light from a smooth surface. In specular reflection, the photons do not interact with the surface at all and so the color of the reflected light is the original color of the incident light. In contrast, a diffuse reflection is produced by rough surfaces. In a diffuse reflection, the light is reflected in all directions and the color of the reflected light depends on the reflection properties of the surface.

Today's ophthalmic microscopes aim to mitigate the problem of Purkinje reflexes by trying to position the reflection in the periphery of the eye lens. However, the best red reflex can be achieved when the eye is illuminated along a light path which is coaxial to the light path of the reflected light used for imaging. The coaxial arrangement of illumination and observation places the reflection in the central area of the eye. Thus, there is a tradeoff between the quality and intensity of the red reflex and the position and strength of the specular reflections.

The present invention seeks to improve microscopes or endoscopes in providing a red reflex of high intensity with minimum or no specular reflections.

According to the invention, this object is achieved for the above-mentioned microscope or endoscope assembly in that the microscope or endoscope assembly comprises an observation region, in which an object to be observed, such as an eye, can be arranged, an illumination light path which extends from an illumination light entry region to the observation region, an observation light path which extends from the observation region to a light exit region, a first polarizing sub-assembly arranged in the illumination light path, a second polarizing sub-assembly arranged in the observation light path, the second polarizing sub-assembly being configured to filter out the polarized light passing the first polarizing sub-assembly, and a beam splitter, which is arranged in the illumination path between the observation region and the light exit region and in the observation light path between the light entry region and the observation region.

The objective is also solved by a method for operating a microscope, wherein the method comprises the steps of generating polarized illumination light, directing the polarized illumination light onto an observation object, where it is reflected, and filtering out the reflected illumination light which has the same polarization as the illumination light.

The microscope or endoscope assembly and the method according to the invention, reduce or eliminate specular reflections using the two complementary polarizing sub-assemblies. The first polarizing sub-assembly polarizes the illumination light directed onto the observed object. In a specular reflection, the polarization of the illumination light is maintained and thus, light in the observation light path, which is generated by specular reflection, is filtered out by the second polarizing sub-assembly. Light in the observation light path which is generated by diffuse reflection has a random polarization and thus may pass at least partly through the second polarizing sub-assembly.

The solution according to the invention may be further improved by the following additional features, which can be independently added independently of one another. As is explained in the following, each of the additional features may be advantageous in itself.

The illumination light enters the microscope sub-assembly at the light entry region. At the light entry region, at least one of a lens and an aperture may be arranged.

The microscope or endoscope assembly may comprise an illumination sub-assembly which may include a light source for generating the illumination light. The first polarizing sub-assembly may also be part of such an illumination sub-assembly if, for example, an illumination sub-assembly is used which generates polarized light for illuminating an object in the observation region.

At the light exit region, the observation light, i.e. the illumination light reflected from an object in the observation region, leaves the microscope or endoscope assembly. At the light exit region, an observation sub-assembly may be located as part of the microscope assembly. The observation sub-assembly is configured for observing an object in the observation area and may comprise at least one of one or more cameras and one or more oculars.

According to another additional feature, the observation path and illumination path may be coaxial to each other at least between the beam splitter and the observation area to maximize the red reflex intensity.

To maintain simplicity of the microscope or endoscope assembly and avoid high costs, the first and second polarizer sub-assemblies may each comprise at least one linear polarizer. The at least one linear polarizer of the first and/or second polarizing sub-assembly may be integrated into the beam splitter so that an overall compact microscope or endoscope assembly is obtained.

The linear polarizer of the first polarizing sub-assembly may be arranged in the illumination light path between the illumination light entry region and the beam splitter, so that this polarizer filters only illumination light.

A linear polarizer of the second polarizing sub-assembly may be arranged in the observation light path between the beam splitter and the light exit region. Thus, the linear polarizer of the second polarizing sub-assembly only filters light reflected from the object.

In another advantageous embodiment, at least one of the first and the second polarizing sub-assembly may comprise a polarization rotor, such as a faraday, birefringent and/or prism rotator. The polarization rotor may in particular be a half or quarter-wave plate. The polarization rotor may, in another embodiment, be part of a circular polarizer, which in turn may be part of at least one of the first polarizing sub-assembly and the second polarizing sub-assembly.

A polarization rotor may be arranged in both the illumination and the observation light path.

The polarization rotor allows adjusting the angle of polarization in the light that passes through it. Thus, it can be used to use a subsequent linear polarizer more efficiently. Further, the use of a polarizing rotor allows to better distinguish between incident, illumination light and reflected, observation light.

The polarization rotor may in particular be located between the beam splitter and the observation region. In such a configuration, the polarization rotor may be used to generate circularly polarized light for illuminating the observation area. Upon specular reflection, the handedness of the circularly polarized light is reversed. Thus, specularly reflected light passing the polarizing rotor, in particular a quarter-wave plate, is linearly polarized in a direction perpendicular to the linear polarization of the illumination light before the polarization rotor.

The first and the second polarizing sub-assemblies may share a common polarization rotor, which may be adapted to nestle to an object in the observation region. This may be achieved by the common polarization rotor having a shape which corresponds at least partly to the shape of the object to be observed. To avoid reflections at the internal surface between the polarization rotor and the object, the refractive indices are matched. Further, there is preferably no air bubble between the polarization rotor and the object due to the snug fit. For example, the polarization rotor may be configured as a contact lens to be applied onto the eye in case of an ophthalmic microscope.

The polarization rotor, in particular, may be a quarter-wave plate. As a contact lens, it may be used to suppress specular reflections from the outermost surface of the object, but retain further specular reflections from internal interfaces of the object such as interfaces between other tissue layers. Thus, information about the three-dimensional structure of these tissue layers, which is contained in the specular reflection, may be maintained.

According to another advantageous embodiment, the beam splitter may be a polarizing beam splitter. The polarizing beam splitter may be part of the first and/or second polarizing sub-assembly or be an additional polarizing device. In such a case, the linear polarizer function of at least one of the first and second polarizing sub-assemblies may be integrated in the beam splitter.

The polarizing beam splitter may be a reflective, birefringent and/or thin-film beam splitter.

For most purposes of this invention, it is sufficient if the beam splitter reflects only light having a predetermined polarization direction and transmits lights without preference of a polarization direction. However, in some applications, a linear polarizer may not be needed if the light which is transmitted through the beam splitter is also linearly polarized.

The polarization direction of the linear polarizer which is facing the reflection side of the beam splitter preferably corresponds to the polarization direction of the light which is reflected by the polarizing beam splitter. The linear polarizer may be part of the first polarizing sub-assembly or the second polarizing sub-assembly, depending on whether the illumination light is reflected by the beam splitter towards the observation area and the reflected light in the observation light path is transmitted through the beam splitter towards the light exit region, or whether the illumination light is transmitted through the beam splitter and the reflected observation light is reflected by the beam splitter.

In one embodiment, linearly polarized illumination light may be incident on the polarizing beam splitter in operation of the microscope assembly. Preferably, the polarization direction of the incident light corresponds to the polarization direction of the light which is reflected by the polarizing beam splitter. In such a configuration, the efficiency of light reflection is increased, as the polarized beam splitter reflects the entire incident illumination light. This compares to a beam splitter without the polarization functionality, which would only reflect half of the incident light. To utilize this efficiency increase, a linear polarizer may be arranged between the light entry region and the polarizing beam splitter. Of course, if the illumination light is to be transmitted through the polarizing beam splitter, then the polarization of the polarized incoming light preferably corresponds to the polarization direction needed for such a transmission.

The notion of polarization direction comprises both the direction in case of linear polarization and in case of circular polarization the handedness of the circularly polarized light.

The efficiency of illumination can be further increased if a further polarizing beam splitter is located in the illumination light path or the observation light path before the polarizing beam splitter, i.e. in the light path of the light incident on the reflection side of the polarizing beam splitter. In particular, the further polarizing beam splitter may be located in the illumination path.

The further polarizing beam splitter may split the illumination or the observation light path into two sub paths, wherein a secondary polarizing sub-assembly may be arranged in each of the sub-paths.

Depending on whether the further polarizing beam splitter is in the illumination light path or in the observation light path, the secondary polarizing sub-assembly may be part of the first or the second polarizing sub-assembly.

Both sub-paths may be directed to either the light exit region or onto the observation area. In one of the sub-paths, the polarizing beam splitter is located. Further, a polarization rotor may be located in at least one of the sub-paths to adapt the polarization direction to the polarization direction needed for total transmission or reflection by the polarizing beam splitter and/or to the polarization direction of the light in the other sub-path. The secondary polarizing sub-assemblies may be used to adjust the polarization direction in each of the sub-paths so that the light in each of the sub-paths has the same polarization.

In one embodiment, a first secondary polarizing sub-assembly may be located between the further polarizing beam splitter and the observation region, whereas a second polarizing sub-assembly is located between the further polarizing beam splitter and the polarizing beam splitter.

At least one of the first and second polarizing sub-assemblies may comprise a polarization rotor. The polarization rotor may be arranged such that there is a linear polarizer located between the polarization rotor and the polarizing beam splitter and/or that the polarization rotor is arranged between the further polarizing beam splitter and a linear polarizer.

Preferably, the illumination in each sub-path is linearly polarized. The second polarizing sub-assembly is used to adjust the polarization direction to the polarization direction of the polarizing beam splitter so that all the light incident on the polarizing beam splitter from the second secondary polarizing sub-assembly is reflected.

The invention also relates to a retrofit or upgrade kit for installation in an existing microscope or endoscope, in particular an existing surgical microscope, such as an ophthalmic microscope. The upgrade kit comprises at least two devices for linear polarization of light and at least one beam splitter, whereby the beam splitter may be one of the devices for linear polarization. The upgrade kit is configured to generate a microscope assembly in any one of the above embodiments. For example, the retrofit kit may have a polarizing beam splitter which replaces an existing non-polarizing beam splitter in the microscope to be retrofitted. The polarization devices may be polarizers which are adapted to be mounted to existing lenses used for illumination and/or observation of the observation area. The retrofit kit, according to one aspect, may also comprise a polarization rotor which is adapted to nestle to an object to be observed and thus suppress or eliminate specular reflections at the first surface of the object.

The invention finally relates to the use of a microscope assembly according to any one of the above embodiments in a microscope, in particular a surgical microscope such as an ophthalmic microscope or in a surgical endoscope for the reduction and/or elimination of specular reflections.

In the following, various embodiments of the invention are described with reference to the accompanying drawings. The embodiments are for illustrative purposes only and in no way should be construed to limit the scope of the invention to what is shown or described in the embodiments. The various features of the embodiments can be combined as has been explained above. In particular, a feature from one embodiment can be added to another embodiment if the technical effect of this feature is needed for a particular application of the invention. Conversely, a feature may be omitted from an embodiment, if the technical effect of this feature is not needed for a particular application of the invention.

In the following, the same reference numerals are used throughout the drawings for elements, which correspond to each other with respect to function and/or design.

In the drawings
- Fig. 1: shows a schematic representation of a microscope or endoscope assembly according to the invention;
- Fig. 2: shows a schematic representation of another microscope or endoscope assembly according to the invention;
- Fig. 3: shows a schematic representation of another microscope or endoscope assembly according to the invention; and
- Fig. 4: shows a schematic representation of another microscope or endoscope assembly according to the invention.

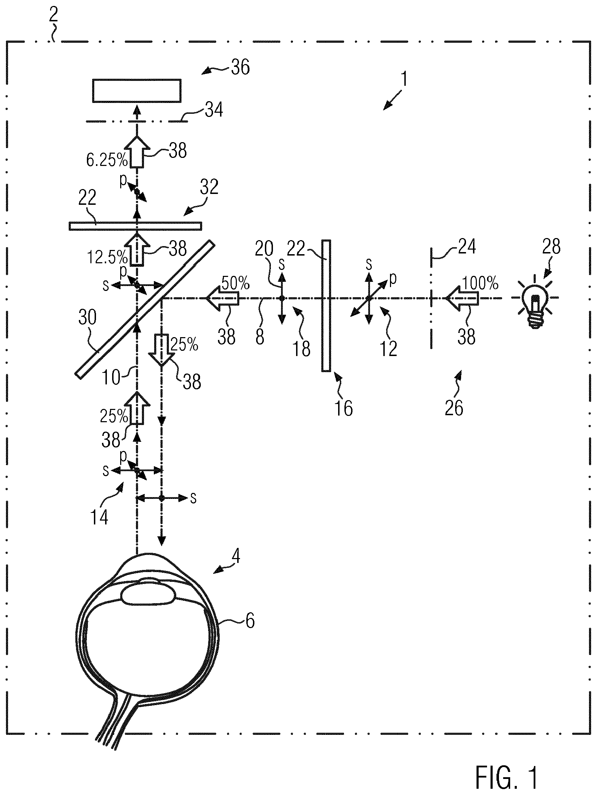

First, the configuration of a microscope or endoscope assembly 1 according to the invention will be explained with reference to Fig. 1.

The microscope and endoscope assembly 1, in short "microscope assembly" in the following, is adapted to be part of or to be installed in a surgical microscope or endoscope 2, in particular an ophthalmic microscope, without being limited to such an application. The microscope or endoscope 2 is shown only schematically and only in Fig. 1.

It is further shown to comprise an observation region 4, in which an object 6 to be observed is placed in operation of the microscope or endoscope 2. The observation region 4 may be part of the microscope or endoscope assembly 1. Although the observation region 4 may not be a structural part of the microscope assembly 1 or the microscope or endoscope 2, it is nonetheless a functional part at least in operation, as the technical effects of the components of the microscope assembly 1 or the microscope or endoscope 2 aim to improve for a great part the imaging of the object 6 to be observed in the observation region 4.

The microscope assembly 1 comprises an illumination light path 8 and an observation light path 10. The illumination light path 8 is the path along which illumination light 12 is directed towards the observation region 4 or, more specifically, onto the object 6 to be observed. In Fig. 1, light in general is schematically depicted by the p and s polarization vectors.

The observation light path 10 is defined by observation light 14. The observation light 14 is light, for the greatest part or exclusively illumination light 12, which is reflected off the object 6.

In the illumination light path 8, a first polarizing subassembly 16 is arranged. The first polarizing subassembly 16 generates polarized light 18 having a polarization direction 20 from the observation light 14 incident onto the first polarizing subassembly 16. The polarization direction 20 may be, as exemplarily shown in Fig. 1, linear or circular. To generate linearly polarized light 18, the first polarizing subassembly 16 may comprise a linear polarizer 22. For generating circularly polarized light 18, the first polarizing subassembly 16 may comprise a circular polarizer (not shown). To form such a circular polarizer, a polarization rotor, such as a quarter-wave plate (not shown) may simply be arranged behind the linear polarizer 22, i.e. on the side of the linear polarizer 22 which faces away from the incident illumination light 12.

The illumination light 12 enters the microscope assembly 1 at a light entry region 24. An illumination subassembly 26 comprising one or more light sources 28 may be located at the light entrance region 24 and be included in the microscope assembly 1 and/or the microscope or endoscope 2. Such an inclusion may e.g. occur if the illumination subassembly already generates polarized light 18, i.e. the first polarizing subassembly 16 is included in the illumination light path 8 in the illumination subassembly 26.

The microscope assembly 1 further comprises a beam splitter 30 which is located both in the illumination light path 8 and the observation light path 10. At least one of the illumination light path 8 and the observation light path 10 is deflected by the beam splitter 30 by reflection. Between the beam splitter 30 and the observation region 4, the illumination light path 8 and the observation light path 10 preferably are arranged coaxially to one another. The beam splitter 30 preferably is a broadband beam splitter.

In Fig. 1 and the remaining embodiments, it is only for illustrative purposes, that the polar light 18 from the first polarization subassembly 16 is reflected at beam splitter 30, i.e. the first polarizing subassembly 22 is located between the light entry region 24 and the beam splitter 30. The reflected light 14 passes through the beam splitter 30. This configuration can also be reversed in that the light entering the microscope assembly 1 at the light entry region 24 passes the beam splitter 30 and the light 14 reflected off the observed object 6 is reflected at the beam splitter 30.

In the observation light path 10, a second polarizing subassembly 32 is located. The configuration of the second polarizing subassembly 32 may be the same as for the first polarizing subassembly 16, except that the filtering function is complementary, i.e. that the second polarizing subassembly 32 filters out light having the polarization direction 18 which is allowed to pass the first polarizing subassembly 16.

For example, the second polarizing subassembly 32 may also comprise a linear polarizer 22 which blocks the passage of light having the polarization direction 20 which allowed it to pass (or is generated by) the linear polarizer 22 of the first polarizing subassembly 16.

The second polarizing subassembly 32 may be located between the beam splitter 30 and a light exit region 34. At the light exit region 34, the observation light 14 may leave the microscope assembly 1. In particular, at the light exit region 34 an observation subassembly 36 such as one or more cameras or one or more oculars may be located.

In the following, the function of the microscope assembly 1 of Fig. 1 is explained.

The polarized illumination light 20 hitting the object 6 undergoes both specular and diffuse reflection at the object 6. The specular reflection will maintain the polarization direction 20 of the observation light, the polarized illumination light 20 which undergoes diffuse reflection will have a random polarization which may deviate from the polarization direction of the incident illumination light. Thus, in addition to the pre-defined polarization direction 20, denoted by s in Fig. 1, imparted by the first polarizing subassembly 16, a polarization direction p orthogonal to the polarization direction 20 will be added to the observation light 10 due to diffuse reflection. Both the diffusedly and the specularly reflected light will pass through the beam splitter 30 and will be filtered out by the second polarizing subassembly 32. Thus, at the light exit region 34, or at the observation subassembly 36 respectively, only light having polarization direction p, i.e. observation light 14 not having the polarization direction 20, s, will be observed. This, in turn, means that all specular reflection will be suppressed or eliminated by the second polarizing subassembly 32.

Thickset arrows 38 indicate both the direction of the light along the illumination light path 8 and the observation light path 10 and its relative intensity compared to the intensity at the light entry region 24.

Assuming equal distribution between the two orthogonal polarization directions p, s, the light intensity after exiting the first polarizing subassembly 16 will be decreased by half compared to the intensity of the incoming illumination light, as half of the light will be filtered out. Another half of the light is lost at each passing, both reflection and transmission, of the beam splitter 30, as the beam splitter 30 reflects half of the light and transmits the other half of light. Finally, again half of the light is lost at the second polarizing subassembly 32, as one polarization direction 20 is filtered out. Thus, assuming ideal conditions and disregarding any impurities and additional losses, only 6.25 % of the incident illumination light entering at the light entry region 24 will be collected at the light exit region 34.

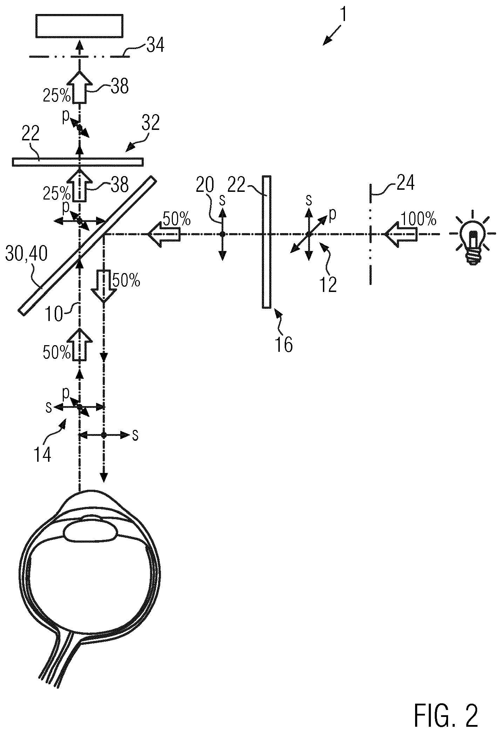

A simple measure is able to quadruple the light output at the light exit region 34, as is shown exemplarily in Fig. 2. For the sake of simplicity, only the differences to the embodiment of Fig. 1 are explained.

In Fig. 2, the set-up of the microscope assembly 1 is the same as in Fig. 1 except that the beam splitter 30 is a polarizing beam splitter 40. The polarizing beam splitter 40 reflects completely light having a polarization direction s. The remaining light having different polarization is transmitted through the polarizing beam splitter 40.

The polarization direction s, which is reflected completely by the beam splitter 40, corresponds to the polarization direction 20 which is imparted to the observation light 20 by the first polarizing subassembly 16 or the linear polarizer 22, respectively. Thus, all the observation light 18 exiting from the first polarizing subassembly 16 is reflected by the beam splitter 40. Of the observation light 14 which is passing through the polarizing beam splitter 40, only light of the orthogonal polarization direction p is transmitted, observation light 14 having the orthogonal polarization direction s is reflected. As this polarization is allowed to pass through the second polarizing subassembly 32 or its linear polarizer 22, respectively, there will be no losses at the second polarizing subassembly 32. As indicated by the arrow 38, 25 % of the light passing through the light entry region 24 will exit at the light exit region 34.

As can be further seen from Fig. 2, the two linear polarizers 22 can be omitted, as the polarization function of the linear polarizer 22 of the first polarizing subassembly 16 can be performed by the polarizing beam splitter 40. The same is true for the linear polarizer 22 of the second polarizing subassembly 32, as the polarizing function in the observation light path 10 now also located on the polarizing beam splitter 40. Thus, the first polarizing subassembly 22 and the second polarizing subassembly 32 both now can be located on the polarizing beam splitter 40.

To further increase the yield at the light exit region 34, the microscope assembly 1 of Fig. 2 can be further modified. The modification includes adding a further polarizing beam splitter 42 in the illumination light path 8 at a location between the light entry region 24 and the polarizing beam splitter 40. The polarizing beam splitter 42 splits the illumination light path 8 into two sub-paths 8a and 8b. One of the sub-paths 8a, 8b, here, light path 8b, is directed towards the polarizing beam splitter 40, where it is transmitted or, as here, reflected. The other light path 8a is directed directly towards the observation region 4 or the observed object 6, respectively.

All light reflected off the further polarizing beam splitter 42, i.e. the observation light 12 in the respective sub-path, here 8a, will be linearly polarized. Between the further beam splitter 42 and the observation region 4 in the path of the observation light 12 reflected off the further beam splitter 42, a linear polarizer 22 may be located to filter out any residual light or different polarization. To keep the set-up inexpensive and easy to maintain to be set-up, the linear polarizer 22 in the light path 8a may also be omitted.

In order to efficiently suppress any specular reflections from the observation area 4 at the light exit region 4, it is beneficial if the polarization direction 20 imparted by the further polarizing beam splitter 42 corresponds to the polarization direction s which is filtered out of the observation light by the polarizing beam splitter 40. In other words, the polarization direction in the sub-paths 8a, 8b should be the same before the illumination light enters the observation region 4. To ensure this, a polarization rotor 44 may need to be inserted into the illumination light path 8, or one of its sub-paths 8a, 8b, respectively.

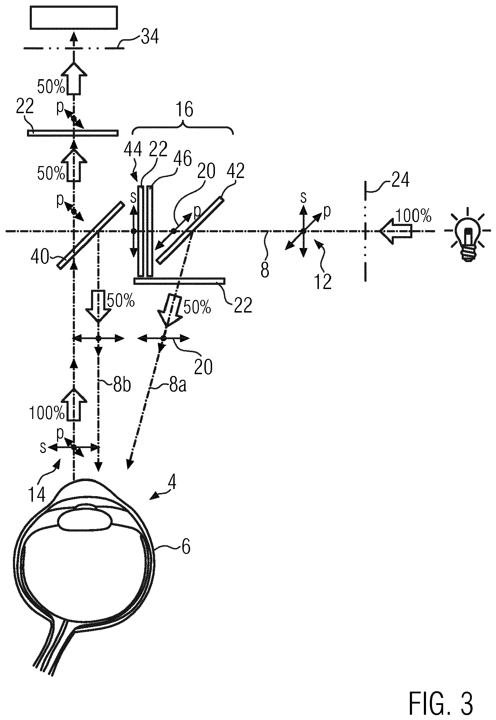

This is explained in the following with exemplary reference to sub-path 8b, where the polarization rotor 44 is located between the further polarizing beam splitter 42 and the polarizing beam splitter 40 in order to rotate the polarization direction 20 of the illumination light 12 after having passed the further polarizing beam splitter 42. I.e. the polarization direction p is rotated to the polarization direction s, which then is reflected entirely by the beam splitter 40 and which corresponds to the polarization direction 20 of the illumination light 12 reflected by the further polarization beam splitter 42 in the other sub-path. The polarization rotor 44 may consist of a half-wave plate 46 and a linear polarizer 22 behind the half-wave plate 46, i.e. at the side of the half-wave plate 46 facing away from the light entry region 24. Of course, if the polarizing beam splitter 42 has a different polarization direction than shown in Fig. 3, a polarization rotor 44 may be located in sub-path 8a instead of sub-path 8b, or in both sub-paths 8a, 8b.

Due to the use of the further polarizing beam splitter 42 and the polarization rotor 44, all of the illumination light 12 can be reflected by the polarizing beam splitter 40, doubling the available light intensity at the light exit region 34.

The general set-up of having the polarization function of the first polarizing subassembly in the illumination light path 8 and the polarization function of the second polarization subassembly 32 in the observation light path 10, and having a polarizing beam splitter 40 in both the illumination light path 8 and the observation light path 10 can be used to suppress specular reflections at an outermost surface 48 of the object 6.

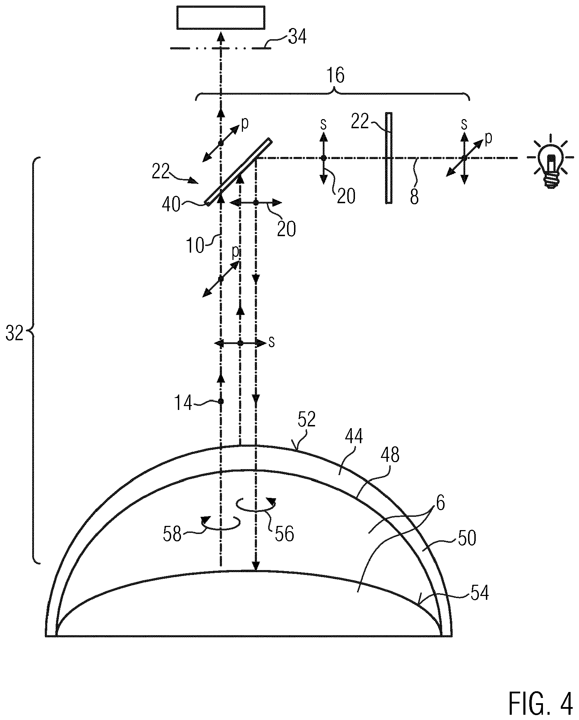

Such a set-up is shown in Fig. 4. *It is particularly useful in ophthalmic microscopes, where the outermost layer of the eye may produce unwanted specular reflections. Again, only differences to the preceding embodiments are explained.

In order to reduce the specular reflection at the outermost surface 48 of the object 6, a polarization rotor 44 will be placed onto the object 6, so that it nestles onto the surface 48. The snug fit ensures tight contact between the polarization rotor and the object without any intermediate bubbles which would cause reflections. If necessary, a fluid may be arranged between the polarization rotor 44 and the object 6 to eliminate air and fill out any gaps.

If the object is an eye, the polarization rotor 44 may be a contact lens 50. The refractive index of the polarization rotor 44 is preferably adapted to the refractive index of the object 6 at least close to the outermost surface 48. Thus, there will be no reflections at the interface between the contact lens 50 and the object 6, however there will be reflections at the outermost surface 52 of the contact lens 50. As the specular reflections maintain the polarization direction 20 imparted by the first polarizing subassembly 16, they will be filtered out by the second polarizing subassembly 32, which in this case is included in the polarizing beam splitter 40, as in the embodiment of Fig. 2.

Specular reflections at a further internal interface 54 are maintained by this set-up in the light arriving at the light exit region 34, so that any three-dimensional information about the structure of this interface 54, which is transported by the specular reflection, is maintained. This is explained by the following.

At the polarizing rotor 44, may e.g. a quarter-wave plate which generates circularly polarized light after having passed through by the illumination light 12. In a specular reflection at the internal interface 54 of the object 6, the polarization direction, i.e. the circular polarization is maintained, only the handedness, i.e. sense of rotation of the circular polarization, is reversed, as can be seen at reference numeral 58.

If the circularly polarized light 58 reflected off the internal surface 54 again passes the polarization rotor 44, it will again be linearly polarized, only shifted in its polarization direction with respect to the polarization direction 20 of the illumination light 12 after having passed the first polarizing subassembly 16. The relative rotation of the linear polarization direction 20, here s, of the polarized illumination light 12, and the polarization direction, here p, of the light resulting from specular reflection at the internal surface 54, results in the observation light 14 being able to pass the polarizing beam splitter 40 unfiltered and proceed to the light exit region 34.

Any diffuse reflection within the object 6 will also result in light having the same polarization direction p as the light reflected specularly by the internal surface 54 and thus will also reach the light exit region 34.

In the case of Fig. 4, both the first and the second polarizing subassembly 16, 32 in effect constitute a circular polarizer of which both components are simultaneously located in the illumination light path 8 and the observation light path 10 and where the linear polarizer of both polarizing subassemblies 16, 32 is integrated in the beam splitter being a polarizing beam splitter 40. The polarization rotor part of the circular polarizer is served by the polarization rotor 44, which in this case attaches to the outermost surface 48 of the object 6.

It is to be understood that the polarization directions p and s in the preceding description are only meant to designate complementary polarization directions and do not carry any physical meaning as to a polarization direction with respect to any electric or magnetic wave directions.

### Reference Numerals

- 1: microscope or endoscope assembly
- 2: microscope or endoscope
- 4: observation region
- 6: object to be observed
- 8: illumination light path
- 8a: sub-path
- 8b: sub-path
- 10: observation light path
- 12: illumination light
- 14: observation light
- 16: first polarizing subassembly
- 18: polarized light
- 20: polarization direction
- 22: linear polarizer
- 24: light entry region
- 26: illumination subassembly
- 28: light source
- 30: beam splitter
- 32: second polarizing subassembly
- 34: light exit region
- 36: observation subassembly
- 38: arrow
- 40: polarizing beam splitter
- 42: further polarizing beam splitter
- 44: polarization rotor
- 46: half-wave plate
- 48: outermost surface of object
- 50: contact lens
- 52: outer surface of contact lens
- 54: internal interface within object
- 56: circularly polarized light
- 58: reflected circularly polarized light

## Claims

1. Microscope or endoscope assembly (1), in particular for a surgical microscope (2) such as an ophthalmic microscope, comprising:
an observation region (4), in which an object (6) to be observed can be arranged,
an illumination light path (8) which extends from an illumination light entry region (24) to the observation region (4), an observation light path (10), which extends from the observation region (4) to a light exit region (34),
a first polarizing subassembly (16) arranged in the illumination light path (8),
a second polarizing subassembly (32) arranged in the observation light path (10), the second polarizing subassembly (32) arranged in the observation light path (10), the second polarizing subassembly (32) being configured to filter out polarized light (18) passing the first polarizing subassembly (16) and a beam splitter (30, 40), which is arranged in the illumination light path (8) between the observation region (4) and the light entry region (24) and in the observation light path (10) between the light exit region (34) and the observation region (4).

2. Microscope or endoscope assembly (1) according to claim 1, wherein the first and second polarizer subassemblies (16, 32) each comprise at least one linear polarizer (22).

3. Microscope or endoscope assembly (1) according to claim 1 or 2, wherein at least one of the first and the second polarizing subassembly (16, 32) comprises a polarization rotor (44).

4. Microscope or endoscope assembly (1) according to any one of claims 1 to 3, wherein the first and the second polarizing subassembly (16, 32) share a common polarization rotor (44).

5. Microscope or endoscope assembly (1) according to claim 3 or 4, wherein the polarization rotor (44) is adapted to be attached to an object (6) to be observed in the observation region (4).

6. Microscope or endoscope assembly (1) according to claim 2, wherein the linear polarizer (22) is integrated into the beam splitter (40).

7. Microscope or endoscope assembly (1) according to any one of claims 1 to 6, wherein the beam splitter (40) is a polarizing beam splitter.

8. Microscope or endoscope assembly (1) according to claim 7, wherein, in operation of the microscope or endoscope assembly (1), linearly polarized illumination light is incident on the polarizing beam splitter (40), the polarization direction (20) of the incident polarized illumination light 18 corresponding to the polarization direction (20) of the light reflected by the polarizing beam splitter (40).

9. Microscope or endoscope assembly (1) according to claim 7 or 8, wherein at least one further polarizing beam splitter (42) is located in the illumination path (8).

10. Microscope or endoscope assembly (1) according to claim 9, wherein the further polarizing beam splitter (42) is located between the polarizing beam splitter (40) and the illumination light entry region (24).

11. Microscope or endoscope assembly (1) according to claim 9 or 10, wherein the further polarizing beam splitter (42) splits the illumination light path (8) into two sub-paths (8a, 8b), and wherein there is a polarization rotor (44) arranged in at least one of the sub-paths (8a, 8b).

12. Microscope or endoscope assembly (1) according to claim 11, wherein the polarization direction (20) of the illumination light (12) behind the first polarizing subassembly (16) is the same in both sub-paths (8a, 8b).

13. Upgrade kit for generating a microscope or endoscope assembly (1) according to any one of claims 1 to 12 in a microscope or endoscope (2), in particular a surgical microscope (2) such as an ophthalmic microscope, the upgrade kit comprising at least two devices (16, 32) for linear polarization of light (12, 14) and at least one beam splitter (30, 40).

14. Use of a microscope or endoscope assembly (1) according to any one of claims 1 to 12 in a microscope or endoscope (2), in particular a surgical microscope (2) such as an ophthalmic microscope, for the reduction and/or elimination of specular reflections.

15. Method for operating an endoscope or a microscope (2), in particular surgical microscope such as an ophthalmic microscope, comprising the steps of generating polarized illumination light (18), directing the polarized illumination light onto an observation object (6), where it is reflected and filtering out the reflected polarized illumination light which has the same polarization direction (20) as the polarized illumination light (18).
